# EUROPEAN PATENT SPECIFICATION

(11) **EP 1 872 849 B1**
(45) Date of publication and mention of the grant of the patent: **07.12.2016**
(21) Application number: 06730591.2
(22) Date of filing: 30.03.2006
(51) Int. Cl.: B01J 13/00, A61K 8/06, A61Q 19/00, B01F 17/00

(54) **ALCOHOL-RESISTANT EMULSION AND PROCESS FOR PRODUCING THE SAME**
ALKOHOLBESTÄNDIGE EMULSION UND HERSTELLUNGSVERFAHREN DAFÜR
ÉMULSION RÉSISTANT AUX ALCOOLS ET PROCÉDÉ SERVANT À PRODUIRE CELLE-CI

(30) Priority: 31.03.2005 JP 2005101043
(43) Date of publication of application: 02.01.2008
(73) Proprietor: Miyazaki Prefecture, Miyazaki-shi, Miyazaki 8808501 (JP)
(72) Inventor: SHIMIZU, Masataka, Miyazaki-shi, Miyazaki 8800303 (JP); TORIGOE, Kiyoshi, Miyazaki-shi, Miyazaki 8800303 (JP); NISHIKATA, Naoko, Miyazaki-shi, Miyazaki 8800303 (JP)
(74) Representative: Vossius & Partner Patentanwälte Rechtsanwälte mbB
(86) International application number: PCT/JP2006/306643
(87) International publication number: WO 2006/106813

(56) References cited:
- EP-A1- 0 779 070
- WO-A1-2004/111168
- JP-A- 09 173 822
- JP-A- 11 189 526
- JP-A- 2001 335 435
- JP-A- 2002 088 243
- JP-A- 2003 155 222
- JP-A- 2005 506 896
- US-A1- 2005 031 653
- US-B1- 6 538 019

## Description

### TECHNICAL FIELD

The present invention relates particularly to a monohydric alcohol-containing O/W emulsion or multiphase emulsion, and a process for producing such emulsions.

### BACKGROUND ART

In technical fields using oil-water emulsions, it is known that when a large amount of amphiphilic organic solvent, such as monohydric alcohols, ketones, etc., is incorporated into an emulsion, the droplets in the emulsion are coalesced so that the emulsion is separated into an oil phase and an aqueous phase (hereinafter referred to as "oil-water two-phase separation). Since an amphiphilic organic solvent is soluble in both oil and aqueous phases, a clear oil-water interface cannot be maintained. More specifically, interfacial orientations of emulsifier molecules, in particular, surfactants are inhibited, so that the presence of such droplets cannot be maintained. As a result of dissolution of an amphiphlic organic solvent in both the oil and aqueous phases, dissolution and precipitation occur freely and largely due to mass transfer to thereby promote oil-water two-phase separation. This series of phenomena can commonly occur. Therefore, it is common technical knowledge that when an oil-water emulsion is to be stably maintained, no amphiphilic organic solvent should be added or only a small amount of amphiphilic organic solvent should be added to minimize any such effect of the amphiphilic organic solvent. Adversely, when a stable oil-water emulsion is expected to be quickly separated into two phases (hereinafter referred to "demulsify" to mean "break the emulsification"), adding a suitable amount of amphiphilic organic solvent is a technique often used.

On the other hand, the need to incorporate such an amphiphilic organic solvent into emulsions has long been present. In particular, the use of ethanol, i.e. the amphiphilic organic solvent safest for living organisms, in various fields, such as the applications of ethanol in foods to make use of its flavor and bactericidal properties; applications in cosmetics to make use of its detergency and permeability; applications in pharmaceuticals to make use of its disinfection capabilities; and applications in chemical products to make use of its solubility can be considered. However, for the reasons stated above, emulsion products containing ethanol have hardly been researched and developed. Emulsion products are produced by incorporating various additives into emulsions. Many of such additives are completely insoluble in water and oils but easily soluble in alcohols. Thus the use of an alcohol enables such additives to be stably contained together in an emulsion. Furthermore, the use of an alcohol can reduce the particle size of the dispersed droplets by utilizing dissolution of the oil phase in the alcohol or reduction of the interfacial tension, so that production of a fine particle size emulsion that could not have been produced by prior art formulations can be produced. In addition to single emulsions such as O/W emulsions, if multiphase emulsions which are stable even in the presence of a high concentration of alcohol (hereinafter referred to as "with tolerance to alcohol") could be obtained, the possibilities of technical development are expected to be further increased.

Examples of oil-water emulsions include O/W emulsions in which oil phase droplets are dispersed in an external aqueous phase; W/O emulsions in which aqueous phase droplets are dispersed in an oil phase; W/O/W emulsions in which oil phase droplets in which internal aqueous phase droplets are encapsulated are dispersed in an external aqueous phase; and O/W/O emulsions in which aqueous phase droplets in which internal oil phase droplets are dispersed are dispersed in an external oil phase. In recent years, an S/O/W emulsion in which solid fine particles are encapsulated in oil phase droplets has been proposed (Japanese Patent Application No. 2002-162072). However, the majority of emulsion products are O/W emulsions and others are W/O emulsions. There are only a few products that are W/O/W emulsions, O/W/O emulsions and S/O/W emulsions, which are also called "multiphase emulsions". In contrast, it is these days, W/O emulsions containing a high concentration of monohydric alcohol in place of an aqueous phase have been developed (Japanese Unexamined Patent Publications Nos. 2001-269115, 2002-332444, 2001-89753, and H11-279572).

As described above, several types of W/O emulsions containing a high concentration of alcohol are known. However, alcohol-containing O/W emulsions, which could find the widest application, have hardly been developed. For example, there are only a few documents reporting on the development of O/W emulsions with tolerance to alcohol (Japanese Unexamined Patent Publications Nos. 2002-348589, 2001-117, and H11-189526)

Japanese Unexamined Patent Publication No. 2001-224955 discloses a method for producing an O/W emulsion, comprising mixing an oil phase comprising an oily component and a nonionic surfactant with an oil phase polyhydric alcohol to form an emulsion comprising the oil phase polyhydric alcohol as a continuous phase, and then adding thereto an aqueous phase comprising an aqueous phase polyhydric alcohol and/or a lower alcohol to provide an emulsion comprising as the continuous phase an aqueous phase containing the oil phase polyhydric alcohol.

However, the above production method is a technique based on the premise that a polyhydric alcohol is present in the continuous phase. In particular, Japanese Unexamined Patent Publication No. 2001-224955 describes in paragraph [0017] that when the amount of polyhydric alcohol is too small, the interfacial tension cannot be reduced, so that a fine droplet size emulsion cannot be produced. That is, it is impossible to know from Japanese Unexamined Patent Publication No. 2001-224955 that a stable emulsion can be produced by using a monohydric alcohol in the absence of a polyhydric alcohol.

JP 2003-155222 A discloses a specific bleaching agent and skin care preparation for bleaching.

EP 0 779 070 A1 discloses a certain oil emulsion containing at least one alkyl polyglycoside.

WO 2004/111168 A1 discloses a surfactant composition comprising at least one branched non-ionic surfactant and at least one surfactant capable of forming liquid crystals in water.

US 2005/031653 A1 discloses specific sprayable O/W emulsions which can be prepared from at least two phases by using a hydrophobic phase comprising gemini surfactants and a hydrophilic phase comprising gemini surfactants with addition of solid particles, a foaming surfactant, or an antitranspirant.

US 6,538,019 B1 describes a certain functional emulsion wherein a material being insoluble or having low solubility with respect to water and oil is dissolved in alcohol and the alcohol is dispersed in oil as dispersed phase.

JP 2001-335435 A discloses a specific liquid hair cosmetic having liquid appearance.

### DISCLOSURE OF THE INVENTION

### Problem to be solved by the Invention

However, the above-mentioned prior art emulsions with tolerance to alcohol do not contain a sufficiently high concentration of alcohol. Furthermore, industrially important ethanol cannot be incorporated into any of the above emulsions. Furthermore, the stability properties of those emulsions, such as a sufficient length of stability for practical use or reliable stability, have not been proved. Thus there is room for improvement, at least in stability.

As described above, O/W emulsions stable in the presence of ethanol, etc. are being researched. However, an alcohol-containing emulsion suitable for practical use has yet to be developed.

Thus, a primary object of the present invention is to provide a monohydric alcohol-containing emulsion that is more stable than prior art emulsions.

### Means for Solving the Problem

A primary object of the present invention is to provide an alcohol-containing emulsion. In view of the above-mentioned problems of the prior art, the present inventors conducted extensive research. As a result, the inventors found that the above object can be achieved by producing a monohydric alcohol-containing emulsion in the presence of a specific emulsifier according to specific production steps, and have accomplished the present invention.

Thus the present invention provides the following emulsions with tolerance to alcohol and production processes thereof.
1. An emulsion with tolerance to alcohol comprising a monohydric alcohol; and
   an emulsifier;
   (1) the content of the monohydric alcohol
      being 40 to 80 wt% or more, based on the total weight of the emulsion;
   (2) the content of the emulsifier being 0.1 to 50 wt%, based on the total weight of the emulsion; and
   (3) the emulsion being in the form of an O/W emulsion, W/O/W emulsion, O/W/O emulsion, or S/O/W emulsion, wherein the emulsifier comprises at least one emulsifier selected from the group consisting of 1) polyoxyethylene hydrogenated castor oils, and 2) polyoxyethylene castor oils.
2. The emulsion with tolerance to alcohol according to Item 1, wherein changes in three variable droplet size values corresponding to 10%. 50% and 90% of a cumulative droplet volume distribution as determined by the laser diffraction/scattering particle size distribution analyzer are within 30%, when comparing the values 1 week after the preparation of the emulsion with tolerance to alcohol to those immediately after the preparation.
3. The emulsion with tolerance to alcohol according to Item 1, wherein the emulsifier comprises an emulsifier having an oil-water distribution coefficient of 0.1 to 10.
4. The emulsion with tolerance to alcohol according to Item 1 wherein the monohydric alcohol accounts for 99.99 to 100 wt% of the total alcohol content of the emulsion with tolerance to alcohol.
5. A process for preparing an emulsion with tolerance to alcohol comprising: (1) a first step of adding an emulsifier to at least either of an aqueous phase starting material or an oil phase starting material; (2) a second step of mixing the aqueous phase starting material and the oil phase starting material to form a precursor emulsion; and (3) a third step of mixing the precursor emulsion with a monohydric alcohol,wherein the content of the monohydric alcohol is 40 to 80 wt% based on the total weight of the emulsion, and wherein the emulsifier comprises at least one emulsifier selected from the group consisting of 1) polyoxyethylene hydrogenated castor oils, and 2) polyoxyethylene castor oils.
6. The process according to Item 5, wherein the emulsifier comprises an emulsifier having an oil-water distribution coefficient of 0.1 to 10.
7. The process according to Item 5, further comprising a step of removing the alcohol from the emulsion obtained in step (3).
8. The process according to Item 5, further comprising a step of forcing the emulsion obtained in step (3) through a porous membrane to reduce the droplet size of the emulsion.

### EFFECT OF THE INVENTION

The emulsion with tolerance to alcohol,
although containing a monohydric alcohol such as ethanol, etc. can be stably maintained in the form of an emulsion over a long period of time. The emulsion with tolerance to alcohol meets a long-standing need to incorporate a monohydric alcohol such as ethanol into emulsions, and is expected to be applicable to various fields, such as foods, cosmetics, pharmaceuticals, chemicals, etc. The production process produces a monohydric alcohol-containing emulsion, so that substances which are completely insoluble in water and oils but soluble in an alcohol can be stably contained in the emulsion. In particular, the production process can reduce the droplet size of emulsion by dissolution in an alcohol, reduction of interfacial tension, etc. Thus, it
can be expected as a fundamental technology that can create many new techniques, such as the production of fine droplet size emulsions that could not have been produced by the prior art.

### BRIEF DESCRIPTION OF THE DRAWINGS

FIG.1 is a conceptual diagram illustrating an O/W emulsion with tolerance to alcohol.
FIG.2 is a conceptual diagram illustrating multiphase emulsions with tolerance to alcohol. FIG.2 (A) is a schematic diagram illustrating W/O/W emulsion. FIG.2 (B) is a schematic diagram illustrating S/O/W emulsion. FIG.2 (C) is a schematic diagram illustrating O/W/O emulsion.
FIG.3 shows droplet size distribution changes in an O/W emulsion with tolerance to alcohol prepared using the emulsifier "HCO-60".
FIG.4 shows droplet size distribution changes in an O/W emulsion with tolerance to alcohol prepared using the emulsifier "Tween 80".
FIG.5 shows droplet size distribution changes representing emulsion droplet size reduction by addition of an alcohol.
FIG.6 is a graph showing droplet size distribution changes representing the six-month stability of an emulsion.
FIG.7 is a graph showing oil phase droplet size changes in an O/W emulsion containing ethanol at a concentration of 69.9 wt% (HCO-50 + Lp oil phase / HCO-60 + poloxamer + aqueous glucose solution).
FIG.8 is a graph showing oil phase droplet size changes in an O/W emulsion containing ethanol at a concentration of 70.1 wt% (HCO-40 + soybean oil phase / HCO-60 + poloxamer + aqueous glucose solution).
FIG.9 is a graph showing oil phase droplet size changes in an O/W emulsion containing ethanol at a concentration of 69.6 wt% (PGCR + soybean oil phase / HCO-60 + poloxamer + aqueous glucose solution).
FIG.10 shows the results of optical microscope observation of a W/O/W emulsion containing ethanol at a concentration of 34.3 wt%, as observed 20 hours after the preparation.
FIG.11 shows the results of optical microscope observation of an O/W/O emulsion containing ethanol at a concentration of 11.4 wt%, as observed 1 hour after the preparation.

### Description of Notations

(1) Oil phase droplet;
(2) External aqueous phase;
(3) Oil phase/external aqueous phase interface;
(4) Internal aqueous phase droplet;
(5) Oil phase droplet;
(6) Internal aqueous phase/oil phase interface;
(7) Oil phase/external aqueous phase interface;
(8) Solid fine particle,
(9) Solid fine particle/oil phase interface;
(10) Internal oil phase droplet;
(11) Aqueous phase droplet;
(12) External oil phase;
(13) Internal oil phase/aqueous phase interface;
(14) Aqueous phase/external oil phase interface;
(15) Oil phase droplet size distribution in an ethanol-free O/W emulsion containing "HCO-60";
(16) Oil phase droplet size distribution in the emulsion of (15) 1 hour after the addition of ethanol to a concentration of 35 wt%;
(17) Oil phase droplet size distribution in the emulsion of (16) 7 days after the addition of ethanol;
(18) Oil phase droplet size distribution in an ethanol-free O/W emulsion containing "Tween 80";
(19) Oil phase droplet size distribution in the emulsion (18) immediately after the addition of ethanol to a concentration of 35 wt%;
(20) Droplet size distribution in an O/W emulsion prepared using a homomixer;
(21) Droplet size distribution in an O/W emulsion prepared by adding ethanol to a concentration of 43.1 wt%, followed by re-emulsification using a homomixer;
(22) Droplet size distribution in an emulsion obtained by subjecting the emulsion of (21) to the membrane permeation;
(23) Droplet size distribution in an ethanol-free emulsion after 6 months;
(24) Droplet size distribution in an emulsion containing ethanol at a concentration of 43.1 wt% after 6 months;
(25) Changes in 10%D of an emulsion containing ethanol at a concentration of 69.9 wt%;
(26) Changes in 50%D of an emulsion containing ethanol at a concentration of 69.9 wt%;
(27) Changes in 90%D of an emulsion containing ethanol at a concentration of 69.9 wt%;
(28) Changes in 10%D of an emulsion prepared not using ethanol;
(29) Changes in 50%D of an emulsion prepared not using ethanol;
(30) Changes in 90%D of an emulsion prepared not using ethanol;
(31) Changes in 10%D of an emulsion containing ethanol at a concentration of 70.1 wt%;
(32) Changes in 50%D of an emulsion containing ethanol at a concentration of 70.1 wt%;
(33) Changes in 90%D of an emulsion containing ethanol at a concentration of 70.1 wt%;
(34) Changes in 10%D of an emulsion prepared not using ethanol;
(35) Changes in 50%D of an emulsion prepared not using ethanol;
(36) Changes in 90%D of an emulsion prepared not using ethanol;
(37) Changes in 10%D of an emulsion containing ethanol at a concentration of 69.9 wt%;
(38) Changes in 50.%D of an emulsion containing ethanol at a concentration of 69.9 wt%;
(39) Changes in 90%D of an emulsion containing ethanol at a concentration of 69.9 wt%,
(40) Changes in 10%D of an emulsion prepared not using ethanol;
(41) Changes in 50%D of an emulsion prepared not using ethanol; and
(42) Changes in 90%D of an emulsion prepared not using ethanol.

The monohydric alcohol contained in the emulsion of the invention is not particularly limited and can be suitably selected according to the application, purpose of use, etc. of the emulsion. In particular, alcohols that are highly soluble in both water and oil (amphiphilic alcohols) are preferably used in the present invention. From this viewpoint, alcohols containing 5 or less carbon atoms are preferable. Examples of such alcohols include ethanol, methanol, 1-propanol, 2-propanol, 1-butanol, 2-butanol, isobutanol, etc. At least one alcohol selected from methanol and ethanol is preferably used in the present invention, and ethanol is particularly preferable. The monohydric alcohol preferably accounts for 99.99 to 100 wt% of the total alcohol content of the emulsion with tolerance to alcohol of the invention, and particularly preferably 100 wt% thereof. In other words, the proportion of polyhydric alcohols contained as alcohols in the emulsion is preferably less than 0.01 wt% of the total alcohol, and it is particularly preferable that no polyhydric alcohols (0 wt%) are contained. The monohydric alcohol for use in the present invention is a monohydric alcohol itself.

According to the present invention, the content of monohydric alcohol in the emulsion is 40 to 80 wt%. The use of monohydric alcohol in the above-mentioned proportion can provide the emulsion with good stability. The emulsifier, which is soluble in both oil and aqueous phases, preferably has an oil-water distribution coefficient of 0.1 to 10, and particularly preferably 0.2 to 5. The "oil-water distribution coefficient" is an index indicating whether the target substance is more easily soluble in water than in oil, or more easily soluble in oil than in water, and is defined as the ratio of the solubility in an oil phase to the solubility in an aqueous phase. Although n-octanol is used as the oil phase according to the official definition, the term "oil-water distribution coefficient" is herein used to refer to solubility in the various oil phases used.

At least one emulsifier selected from polyoxyethylene hydrogenated castor oils and polyoxyethylene castor oils are used in the invention. Any emulsifier that has the above-mentioned oil-water distribution coefficient can be used. For example, one or more emulsifiers selected from polyoxyethylene hydrogenated castor oils, polyoxyethylene castor oils, polyoxyethylene sorbitans, polyglycerin condensed ricinoleic acid esters, sorbitans, polyglycerin fatty acid esters, sucrose fatty acid esters, polyethylene glycol fatty acid esters, lecithins, and anions can be used.

Although the polyoxyethylene hydrogenated castor oil to be used is not particularly limited in the number of moles of ethylene oxide added, the number is preferably 5 to 100. Although the polyoxyethylene castor oil to be used is not particularly limited in the number of moles of ethylene oxide added, the number is preferably 3 to 60. Commercially available products may be used as such emulsifiers.

The content of emulsifier in the emulsion can be suitably selected according to the kind of emulsifier used, and is 0.1 to 50 wt%, and particularly 0.5 to 10 wt%.

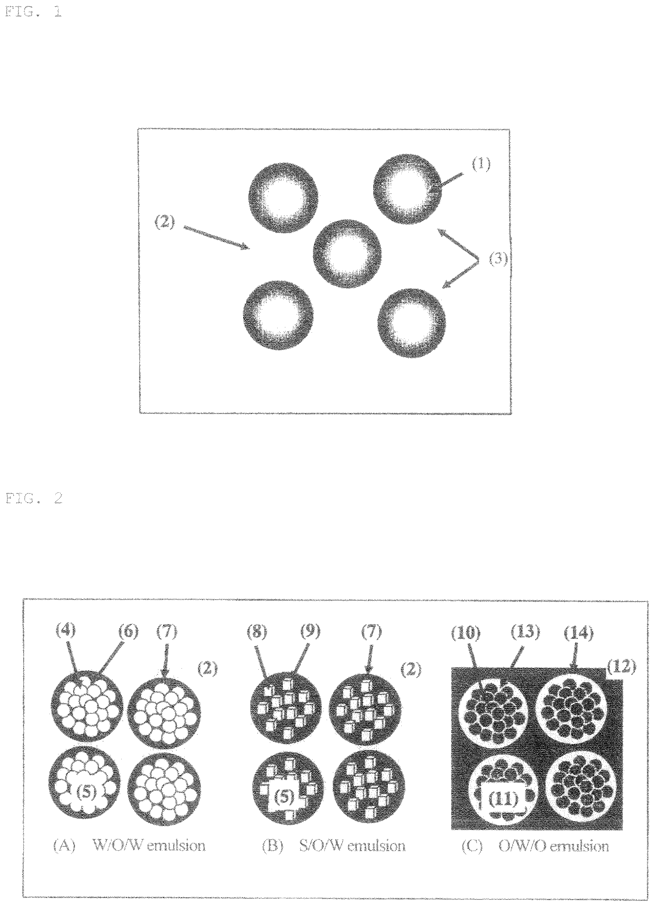

The emulsion with tolerance to alcohol may be in the form of an O/W emulsion as shown in FIG.1 or in the form of multiphase emulsions as shown in FIG.2. The multiphase emulsion may be in the form of a W/O/W emulsion as shown in FIG.2(A), an S/O/W emulsion as shown in FIG.2 (B), or an O/W/O emulsion as shown in FIG.2 (C).

First, the aqueous phase (W), oil phase (O), and solid phase (S) in each form of emulsion are described.

The "aqueous phase" refers to a phase comprising water and an emulsifier, and optionally aqueous additives. The aqueous phase is usually not miscible with an oil phase.

The "oil phase" refers to a phase comprising oil and an emulsifier, and optionally oily additives. The oil phase is usually not miscible with an aqueous phase. The kind and amount of oil used does not substantially affect the tolerance to alcohol. Any oil that is commonly contained in emulsions, such as lipids and hydrocarbons, can be used.

Examples of the "solid phase" include various kinds of pharmaceuticals, foods, cosmetics, coloring agents, etc. Specific examples thereof include anticancer agents such as irinotecan hydrochloride, epirubicin hydrochloride, etc.; reducing agents such as diphenylguanidine bromate, hydrohalic acid salts, etc.; and coloring materials such as methylene blue, rhodamine, etc.

The "O/W emulsion" refers to an emulsion in which oil phase droplets (1) are dispersed in an external aqueous phase (2), and a monohydric alcohol is dissolved in both the oil and external aqueous phases. Although the emulsifier is generally gathered and oriented on the oil phase/external aqueous phase interface (3), a part of the emulsifier is dissolved in both phases.

The "W/O/W emulsion" as shown in FIG.2 (A) is an emulsion in which oil phase droplets (5) in which internal aqueous phase droplets (4) are encapsulated are dispersed in an external aqueous phase (2), and a monohydric alcohol is dissolved in the internal aqueous phase, oil phase and external aqueous phase. Although the emulsifier is generally gathered and oriented on the internal aqueous phase/oil phase interface (6) and on the oil phase/external aqueous phase interface (7), a part of the emulsifier is dissolved in both phases.

The "S/O/W emulsion" as shown in FIG.2 (B) is an emulsion in which oil phase droplets (5) in which solid fine particles (8) are encapsulated are dispersed in an external aqueous phase (2), and a monohydric alcohol is dissolved in both the oil and external aqueous phases. Although the emulsifier is generally gathered and oriented on the solid fine particle/oil phase interface (9) and on the oil phase/external aqueous phase interface (7), a part of the emulsifier is dissolved in the oil phase and the aqueous phase.

The "O/W/O emulsion" as shown in FIG.2 (C) is an emulsion in which aqueous phase droplets (11) in which internal oil phase droplets (10) are dispersed are dispersed in an external oil phase (12), and a monohydric alcohol is dissolved in the internal oil phase, aqueous phase, and external oil phase. Although the emulsifier is generally gathered and oriented on the internal oil phase/aqueous phase interface (13) and on the aqueous phase/outer oil phase interface (14), a part of the emulsifier is dissolved in both phases.

The emulsion, which contains a monohydric alcohol, can be maintained in the form of an emulsion over a comparatively long period of time.

Herein,this characteristic is referred to as "tolerance to alcohol". The emulsion with tolerance to alcohol preferably has the following changes in three variable droplet size values corresponding to 10% of a cumulative droplet volume distribution as determined by the laser diffraction/scattering particle size distribution analyzer (10%D), 50% thereof (50%D) and 90% thereof (90%D),
1) the changes from the values immediately after preparation of the emulsion with tolerance to alcohol to those 1 hour after preparation are within 30% (more preferably 10% or less);
2) preferably, the changes from the values immediately after preparation of the emulsion with tolerance to alcohol to those 24 hours (1 day) after preparation are within 30% (more preferably 10% or less),
3) more preferably, the changes from the values immediately after preparation of the emulsion with tolerance to alcohol to those 3 days after preparation are within 30% (more preferably 10% or less), and
4) most preferably, the changes from those immediately after preparation of the emulsion with tolerance to alcohol to those 7 days after preparation are within 30% (more preferably 10% or less). Of course, emulsions whose changes in the above three variable droplet size values can be maintained within the above-mentioned range for more than 7 days (1 week) (preferably 6 months or more) are included in the scope of the emulsions.

Herein, the droplet size corresponding to 50% of a cumulative droplet volume distribution (50%D) is referred to as "mean particle size" or "mean droplet size", and the size of each droplet is referred to as "particle size" or "droplet size".

Herein, the emulsion stability is clearly defined to characterize the tolerance. More specifically, the emulsion whose changes in the three variable droplet sizes 10%D, 50%D and 90%D of a cumulative droplet volume distribution as determined by the laser diffraction/scattering particle size distribution analyzer are maintained within 30% for 1 week or longer is evaluated as being highly stable and having high tolerance to alcohol.

A laser diffraction/scattering particle size distribution analyzer is herein described as a measurement instrument. This is because the droplet size and droplet distribution determined often vary depending on the measurement technique used, and it is considered necessary to specify the measurement technique. The submicron to micron sized dispersed droplets are too small to be observed under an optical microscope, and are difficult to be directly observe under an electron microscope due to the shape change caused by vacuum drying. Although measurement instruments such as centrifugal sedimentation systems, dynamic scattering systems, static scattering systems, laser diffraction scattering systems, etc. are available, each instrument has its specific characteristics, and the mean droplet size value of the same oil droplet determined often varies depending on the instrument used. In this specification, the laser diffraction/scattering particle size distribution analyzer is selected because it is the apparatus most widely used and the measurement range is as broad as from several tens of nanometers to several hundred micrometers. However, as long as the droplet diameter and droplet distribution changes can be accurately measured, the instrument is not limited to such a laser diffraction/scattering particle size distribution analyzer.

### (2) Process of Preparing the Emulsion with Tolerance to Alcohol

The process for preparing emulsions with tolerance to alcohol is not particularly limited, as long as the emulsion obtained thereby has the above-mentioned characteristics. However, emulsions with tolerance to alcohol
can be advantageously prepared by the following production process.

More specifically, one preferable example of a process for preparing an emulsion with tolerance to alcohol is a process comprising: (1) a first step of adding an emulsifier to at least either of an aqueous phase starting material or an oil phase starting material; (2) a second step of mixing the aqueous phase starting material and the oil phase starting material to form a precursor emulsion; and (3) a third step of mixing the precursor emulsion with a monohydric alcohol.

According to the production process prior to blending an alcohol, an emulsion is prepared and then a predetermined alcohol is added, whereby the size of dispersed droplets can be reduced by utilizing the dissolution of a part of the oil phase in an alcohol, or reduction of interfacial tension, so that a fine droplet size emulsion, that could not have been obtained by prior art emulsification techniques, can be produced. The above method is described below in more detail as representative examples and reference examples of a process for preparing the emulsion with tolerance to alcohol.

### First Step

In the first step, an aqueous phase starting material and an oil phase starting material, at least one of which contains an emulsifier, are prepared. More specifically, starting materials for the aqueous and oil phases of the emulsion, as mentioned above, are prepared. The emulsifier may be selected from those mentioned above. The emulsifier may be incorporated into either one of the aqueous and oil phase starting materials, or both. If necessary, known aqueous or oil additives may be added to the aqueous or oil phase starting material.

When a W/O/W emulsions is expected to be obtained, a W/O emulsion can be used instead of the oil phase starting material in the first step. When an S/O/W emulsion is to be obtained, an S/O suspension can be used instead of the oil phase starting material in the first step. The W/O emulsion and S/O suspension can be prepared in accordance with known methods.

The ratio of the aqueous phase starting material to the oil phase starting material is not particularly limited, and can be suitably selected so that the volume ratio of the aqueous phase to the oil phase in the precursor emulsion obtained in the second step is within the range of 1:0.5 to 1:10000.

### Second Step

In the second step, the aqueous phase starting material and the oil phase starting material are mixed to prepare a precursor emulsion. The mixing method is not particularly limited as long as the desired emulsified state can be obtained. Examples of mixing methods that can be used include stirring emulsification, ultrasonic emulsification, homomixer emulsification, high-pressure homogenizer emulsification, pumping emulsification, membrane emulsification, channel emulsification, etc.

### Third Step

In the third step, a monohydric alcohol is mixed into the emulsion. The monohydric alcohol or the solution thereof may be selected from those mentioned above. The mixing method is not particularly limited. Any stirring method that can dissolve the monohydric alcohol or solution thereof in the aqueous and oil phases may be used.

An O/W emulsion with tolerance to alcohol of the present invention is obtained in the third step. When an O/W/O emulsion is to be prepared, the O/W emulsion is dispersed as a starting material in an oil phase according to a known method to provide an O/W/O emulsion.

An alternative production process comprises preparing an aqueous phase or oil phase starting material containing a specific amount of a monohydric alcohol and then performing the first and second steps to provide an emulsion with tolerance to alcohol. This production process is suitable for producing a low water content emulsion containing a high concentration of alcohol.

In the production process, the alcohol may be removed from the obtained emulsion with tolerance to alcohol, if necessary. The alcohol can be removed from the emulsion with tolerance to alcohol by usual methods, such as distillation with heating, distillation under reduced pressure, and dialysis.

The production process may further comprise a step of forcing the emulsion obtained in the third step through a porous membrane to reduce the droplet size of the emulsion with tolerance to alcohol. The conditions under which the emulsion is forced through the porous membrane may be the same as in a known method. Examples of porous membranes preferable for such use include porous glass membranes, etc. Examples of porous glass membranes preferable for use include those produced by micro-phase separation of glass. Specific examples thereof include CaO-B₂O₃-SiO₂-Al₂O₃ porous glass disclosed in Japanese Patent No. 1504002; CaO-B₂O₃-SiO₂-Al₂O₃-NaO₂ porous glass and CaO-B₂O₃-SiO₂-Al₂O₃-NaO₂-MgO porous glass disclosed in Japanese Patent No. 1518989 and U.S. Patent No. 4657875; and SiO₂-ZrO₂-Al₂O₃-B₂O₃-NaO₂-CaO porous glass disclosed in Japanese Unexamined Patent Publication NO. 2002-160941. Products of porous membrane commercially available can also be used.

### EXAMPLES

Examples, Reference Examples and Comparative Examples are given below to further illustrate the present invention and disclosure, but it is to be understood that the invention is not limited thereto or thereby.

### Reference Example 1

An emulsifier for producing an emulsion with tolerance to alcohol was found in the present invention. To verify this, alcohol-containing emulsions each containing a different type of emulsifier were prepared and evaluated for their stability to investigate the tolerance to alcohol of the emulsifiers.

The emulsifiers listed below in Table 1 were prepared. O/W emulsions containing those emulsifiers were prepared, and evaluated for their stability. Soybean oil (product of Wako Pure Chemical Ind. Ltd.) was used as the oil phase, and a 0.9 wt% aqueous sodium chloride solution was used as the external aqueous phase. The cases of i) adding each emulsifier to the oil phase; and ii) adding each emulsifier to the aqueous phase were investigated, respectively. In case i), while 9.5 g of an oil phase prepared by uniformly mixing 0.5 g of each emulsifier with 9 g of a soybean oil having a specific gravity of 0.9 was added gradually to 19.5 g of an external aqueous phase, the mixture was stirred at 16,000 rpm for emulsification using a homomixer for 1 minute to prepare an O/W emulsion at room temperature. Similarly, in case ii), 0.5 g of each emulsifier was added to 19.5 g of the external aqueous phase. While 9 g of soybean oil was added gradually to the external aqueous phase, the mixture was stirred at 16,000 rpm for emulsification using a homomixer for 1 minute to prepare an O/W emulsion at room temperature.

Subsequently, 8 g of ethanol with a specific gravity of 0.78 was added as a typical monohydric alcohol, and mixed by upsetting repeatedly to prepare the predetermined emulsions. The state of the emulsions was observed 1 hour, 24 hours, and 7 days after the preparation. The ethanol concentration in each O/W emulsion was 21.6 wt%, based on the total weight of each emulsion.

The emulsions thus obtained were evaluated for their stability with the naked eye. The object of this evaluation was to preliminarily screen a large number of emulsifiers to narrow down the emulsifier candidates for the evaluation in Example 2, so only qualitative evaluation with the naked eye was performed according to the following criteria: In either case i) or ii), as compared with the state immediately after the preparation,
A: substantially no change was observed in the emulsion,
B: partial oil-water separation was observed in the emulsion, or a coalescence of oil droplets was clearly observed under a microscope,
C: complete oil-water separation was observed in the emulsion.

Emulsifiers sold by Nikko Chemicals Co., Ltd., Wako Pure Chemical Industries, Ltd., Sakamato Yakuhin Kogo Co., Ltd., Kao Corporation, and Mitsubishikagaku Foods Corporation were used.

Table 1 shows the results. The results show that oil-water separation was not observed in any emulsion and the emulsion state was maintained by all samples. In particular, the emulsions containing a polyoxyethylene hydrogenated castor oil had high stability even in the presence of 21.6 wt% of ethanol, and no substantial change was observed even 7 days after the addition of ethanol.

**Table 1 Screening of Tolerance to Ethanol**

| Emulsifier product name | Manufacturer | Contents | Ethanol concentration: 21.6 wt% | | | | | |
|---|---|---|---|---|---|---|---|---|
| | | | (i) Addition to the oil phase | | | (ii) Addition to the external aqueous phase | | |
| | | | Observed after 1 hour | After 24 hours | After 7 days | Observed after 1 hour | After 24 hours | After 7 days |
| HCO-40 | Nikko Chemicals Co., Ltd. | Polyoxyethylene (40) hydrogenated castor oil | A | A | A | A | A | A |
| HCO-50 | Nikko Chemicals Co., Ltd. | Polyoxyethylene (50) hydrogenated castor oil | A | A | A | A | A | A |
| HCO-60 | Nikko Chemicals Co., Ltd. | Polyoxyethylene (60) hydrogenated castor oil | - | - | - | A | A | A |
| Tween 60 | Wako Pure Chemical Industries, Ltd | Polyoxyethylene (20) sorbitan monostearate | A | A | B | A | B | B |
| Tween 40 | Wako Pure Chemical Industries, Ltd. | Polyoxyethylene (20) sorbitan monopalmitate | A | A | B | A | B | B |
| Tween 20 | Wako Pure Chemical Industries, Ltd. | Polyoxyethylene (20) sorbitan monolaurate | B | B | B | A | B | B |
| MSW-750 | Sakamato Yakuhin KogoCo, Ltd. | Decaglycerol monostearate | A | B | B | A | A | B |
| SWA-10D | Mitsubishika gaku Foods Corporation | Glycerol stearate | - | - | - | A | B | B |
| SWA-15D | Mitsubishika gaku Foods Corporation | Glycerol stearate | - | - | - | A | B | B |
| SWA-20D | Mitsubishika gaku Foods Corporation | Glycerol stearate | - | - | - | A | B | B |

### Reference Example 2

Using the emulsifiers listed below in Table 2, stability was investigated according to the same procedure as in Example 1. In both cases of i) adding each emulsifier to the oil phase and ii) adding each emulsifier to the external aqueous phase, the ethanol concentration in the emulsion was adjusted to 35 wt%, based on the total weight of the emulsion.

The droplet size distribution was measured using a laser diffraction/scattering particle size distribution analyzer ("SALD-2000", product of Shimadzu Corporation), and the O/W emulsions were evaluated for stability from the changes in 10%D, 50%D, 90%D of the cumulative droplet volume distribution. More specifically, the values of the O/W emulsion 1 hour, 3 days, 7 days after the addition of ethanol were compared with those before the addition of ethanol. When the maximum change in 10%D, 50%D, and 90%D was 10% or less, the emulsion was ranked as "A". When the maximum change in wood, 50%D, and 90%D was more than 10% but not more than 30%, the emulsion was ranked as "B". When the maximum change in 10%D, 50%D, and 90%D was more than 30%, the emulsion was ranked as "C". The emulsions ranked "A" were evaluated as having no change in oil phase droplet size; emulsions ranked "B" as having minor change but being satisfactory for practical use and considered stable; emulsions ranked "C" as having oil-water separation and being unstable.

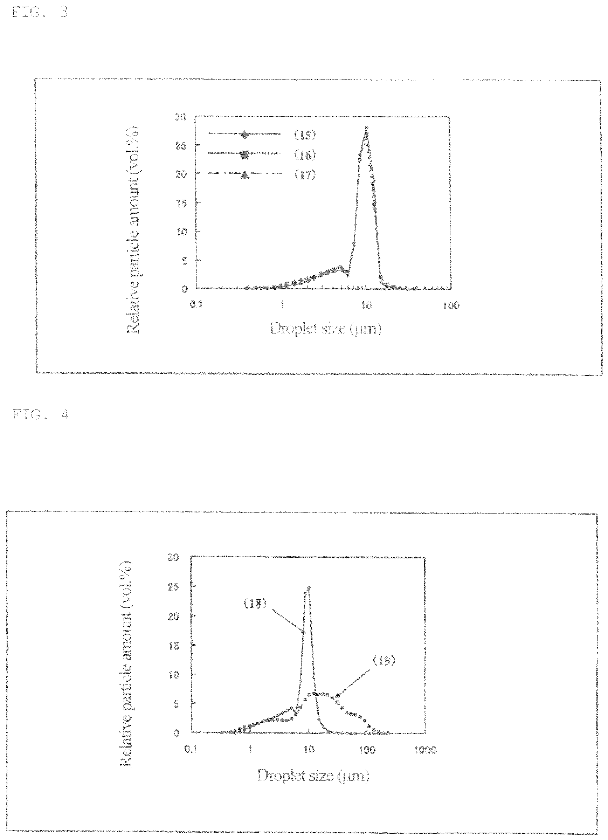

Table 2 shows the results. In all of cases i) and ii), emulsions including the polyoxyethylene hydrogenated castor oils exhibited tolerance to alcohol, and emulsions including the emulsifiers "HCO-20" to " HCO-60" with 20 to 60 moles of ethylene oxide added thereto exhibited particularly remarkable tolerance to alcohol. Likewise, emulsions including the polyoxyethylene castor oils '"CO-3" to "CO-60" with 3 to 60 moles of ethylene oxide added thereto exhibited tolerance to alcohol. FIG.3 shows that when a monohydric alcohol was added to an O/W emulsion containing "HCO-60" with 60 moles of ethylene oxide added thereto, the oil phase droplet size distribution did not change at all even after 7 days. In FIG.3, graph (15) shows the oil phase droplet size distribution of an O/W emulsion prepared not using ethanol; graph (16) shows the oil phase droplet size distribution of this emulsion 1 hour after the addition of ethanol to a concentration of 35 wt%; and graph (17) shows the oil phase droplet size distribution of this emulsion 7 days after the addition of ethanol. The changes in 10%D, 50%D, and 90%D from (15) → (16) → (17) were such that 3.25 µm → 2.60 µm (change: 20%) → 3.11 µm (4.3%) in 10%D; 8.26 µm → 8.34 µm (3.2%) → 8.50 µm (1.4%) in 50%D; and 11.6 µm → 11.6 µm (0%) → 11. 6 µm (0%) in 90%D. The results confirm that this emulsion is highly stable. No change was observed in the emulsion even 1 month after the addition of ethanol.

For comparison, FIG.4 shows droplet size distribution changes in an O/W emulsion with tolerance to alcohol prepared using the emulsifier "Tween 80". In FIG.4, graph (18) shows the oil phase droplet size distribution of an O/W emulsion prepared not using ethanol; and graph (19) shows the oil phase droplet size distribution of this emulsion immediately after the addition of ethanol to a concentration of 35 wt%. A comparison of 10%D, 50%D, and 90%D in graph (19) with those in graph (18) shows the following results: 2.20 µm → 2.01 µm (change: 8.6%) in 10%D; 7.92 µm → 13.4 µm (-69.2%) in 50%D; and 11.2 µm → 54.1 µm (-383%) in 90%D. The results show that significant droplet coalescence occurred in the emulsion immediately after the addition of ethanol to a concentration of 35 wt%, thus lacking tolerance to alcohol.

**Table 2 Test of Tolerance to Ethanol in 35 wt%**

| | Emulsifier | Ethanol | | | | | |
|---|---|---|---|---|---|---|---|
| | | (i) Addition to the oil phase | | | (ii) Addition to the eternal aqueous phase | | |
| | | Observed after 1 hour | After 3 days | After 7 days | Observed after 1 hour | After 3 days | After 7 days |
| 1 | HCO-5 | B | B | B | B | B | B |
| 2 | HCO-10 | B | B | B | B | B | B |
| 3 | HCO-20 | A | B | B | A | A | A |
| 3 | HCO-30 | A | A | A | A | A | A |
| 4 | HCO-40 | A | A | A | A | A | A |
| 5 | HCO-50 | A | B | A | A | A | A |
| 6 | HCO-60 | A | A | A | A | A | A |
| 7 | HCO-80 | A | A | A | B | B | B |
| 8 | HCO-100 | B | B | B | B | B | B |
| 9 | CO-3 | B | B | B | B | B | B |
| 10 | CO-10 | B | B | B | B | B | B |
| 11 | CO-20 | B | B | B | B | B | B |
| 12 | CO-40 | B | B | B | B | B | B |
| 13 | CO-50 | B | B | B | B | B | B |
| 14 | CO-60 | B | B | B | B | B | B |

| | | | | | | | |
|---|---|---|---|---|---|---|---|
| Criteria: A: The maximum change in 10%D, 50%D and 90%D was 10%. or less. B: The maximum change was 10% to 30%. C: The maximum change was more than 30%. | | | | | | | |

In Table 2, "CO-3", "CO-10", "CO-20", "CO-40", "CO-50", and "CO-60" are polyoxyethylene castor oils with 3, 10, 20, 40, 50, and 60 moles of ethylene oxide added thereto, respectively (all manufactured by Nikko Chemicals Co., Ltd.).

Table 2 clearly shows that the emulsions containing at least one emulsifier selected from polyoxyethylene hydrogenated castor oils and polyoxyethylene castor oils have excellent emulsion stability even in the presence of ethanol in a high concentration of 35 wt%; in particular, polyoxyethylene hydrogenated castor oils with 20 to 60 moles of ethylene oxide added thereto achieve particularly excellent effects.

### Reference Example 3

Using various alcohols as monohydric alcohols, emulsion stability was evaluated. More specifically, emulsions were prepared using the emulsifier "HCO-40" under the same conditions as in Example 2, and methanol, ethanol, or propanol was added as a monohydric alcohol to a concentration of 35 wt%. Table 3 shows the results. The results show that the O/W emulsions containing any alcohol were stable for at least 7 days.

**Table 3 Effect of Monohydric Alcohol on Tolerance**

| | Stability of Emulsion | | | | | |
|---|---|---|---|---|---|---|
| | (i) Addition of HCO-40 to the oil phase | | | (ii) Addition of HCO-40 to the external aqueous phase | | |
| Alcohol | Observed after 1 hour | After 3 days | After 7 days | Observed after 1 hour | After 3 days | After 7 days |
| Methanol | A | A | A | A | A | A |
| Ethanol | A | A | A | A | A | A |
| Propanol | A | A | A | A | A | A |

| | | | | | | |
|---|---|---|---|---|---|---|
| Criteria: A: The maximum change in 10%D, 50%D and 90%D was 10% or less. B: The maximum change was 10% to 30%. C: The maximum change was more than 30%. | | | | | | |

### Reference Example 4

The oil phase used in Examples 1 to 3 was soybean oil belonging to vegetable oils, i.e., triglyceride. To confirm that the present invention is applicable to emulsions comprising an oil phase other than triglyceride, emulsions comprising kerosene, liquid paraffin, or toluene, and further comprising ethanol were prepared, and investigated for their stability. The conditions used were the same as in Examples 2 and 3. Table 4 shows the results.

**Table 4 Effect of the Kind of Oils Used on Tolerance**

| | | Stability of emulsion | | | | | |
|---|---|---|---|---|---|---|---|
| | | (i) Addition of emulsifier to the oil phase | | | (ii) Addition of emulsifier to the external aqueous phase | | |
| Kind of oil | Emulsifier | Observed after 1 hour | After 3 days | After 7 days | Observed after 1 hour | After 3 days | After 7 days |
| Soybean oil | HCO-40 | A | A | A | A | A | A |
| | HCO-60 | A | A | A | A | A | A |
| Kerosene | HCO-40 | A | A | A | A | A | A |
| | HCO-60 | A | A | A | A | A | A |
| Liquid paraffin | HCO-40 | A | A | A | A | A | A |
| | HCO-60 | A | A | A | A | A | A |
| Toluene | HCO-40 | A | A | A | A | A | A |
| | HCO-60 | B | B | B | A | A | A |

| | | | | | | | |
|---|---|---|---|---|---|---|---|
| Criteria: A: The maximum change in 10%D, 50%D and 90%D was 10% or less. B: The maximum change was within the range of 10% to 30%. C: The maximum change was more than 30%. | | | | | | | |

The results of Table 4 clearly show that the O/W emulsions with any oil phase were stable for at least 7 days, and the desired stability can be obtained, irrespective of the kind of oil phase used.

### Reference Example 5

The oil-water distribution coefficients of emulsifiers were investigated. More specifically, an equivalent amount of a soybean oil and water were prepared, and an emulsifier was added to a concentration of 5 wt%, based on the total weight thereof. The resulting mixture was stirred under heat to completely dissolve the emulsifier, thus yielding an O/W emulsion. After the emulsion was allowed to stand for 2 days to sufficiently distribute the emulsifier, the aqueous phase was collected by centrifugal filtration. The emulsifier concentration Cw in the aqueous phase was measured using a total organic carbon analyzer ("TOC-500", product of Shimadzu Corporation). The emulsifier concentration Co in the oil phase can be obtained from the amount of emulsifier added. Accordingly, the oil-water distribution coefficient Co/Cw of the emulsifier can be obtained. When polyoxyethylene hydrogenated castor oils ("HCO-10" to "HCO-100") with high tolerance to alcohol were used as emulsifiers, Co/Cw was 0.17 to 9.7. When using "HCO-20" to "HCO-60" with particularly excellent tolerance to alcohol, Co/Cw was 0.3 to 4.9.

### Example 6

This experiment confirmed that the use of an alcohol can reduce the oil phase droplet size of an emulsion and maintain the fine droplet state.

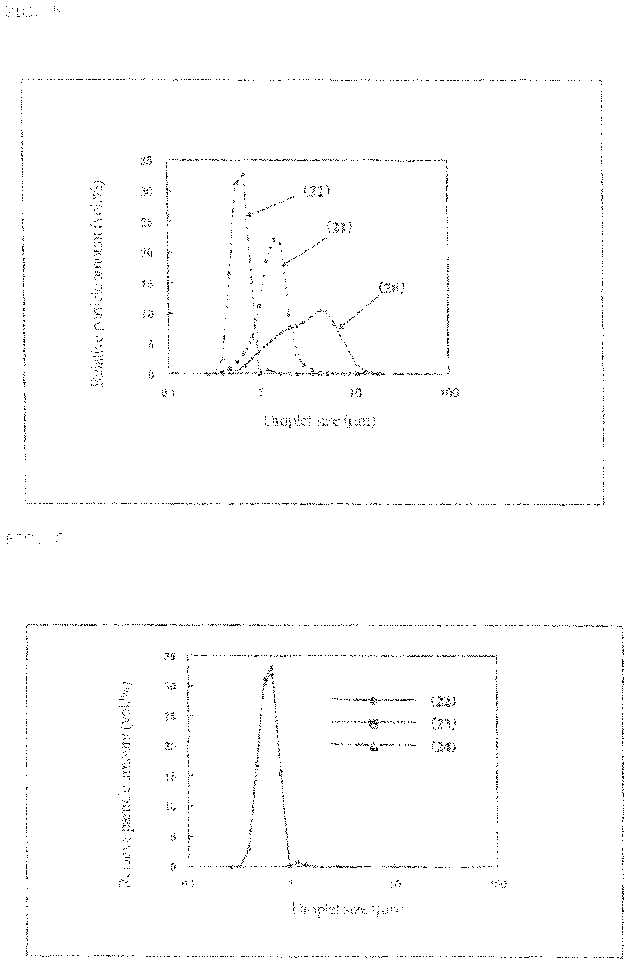

More specifically, 1 g of a hydrogenated castor oil with 40 moles of polyoxyethylene added thereto ("HCO-40", product of Nikko Chemicals) was added to 8 g of a soybean oil to prepare an oil phase. 93 g of a 5 wt% aqueous glucose solution containing 1 g of "HCO-60" was prepared as an external aqueous phase. An O/W emulsion was prepared by stirring at 16,000 rpm using a homomixer for 1 minute. FIG.5 shows the droplet size distribution (20) of the emulsion. After 78 g of ethanol was added to 103 g of the emulsion to achieve an ethanol concentration of 43.1 wt%, the resulting mixture was stirred again using the homomixer. As a result, the droplet size of the emulsion was reduced, and a droplet size distribution (21) that had shifted to a smaller droplet size was obtained. This emulsion was forced through a porous glass membrane with a pore size of 0.5 µm according to the membrane permeation method to yield 180 g of a monodisperse emulsion (22) with a mean droplet size of 550 nm. The results clearly show that the droplet size of the emulsion can be reduced by this process.

A part of the emulsion (22) was placed into a dialysis membrane, and the external aqueous phase was dialyzed to remove ethanol. The ethanol-free emulsion and the ethanol-containing emulsion were preserved in a refrigerator in darkness for 6 months, and the droplet size distribution was measured using the laser diffraction/scattering particle size distribution analyzer ("SALD-2000", product of Shimadzu Corporation). FIG.6 shows the results. The droplet size distribution (23) of the ethanol-free emulsion did not change at all, and the mean droplet size was 559 nm. The droplet size distribution (24) of the ethanol-containing. emulsion did not change, either, and the mean droplet size was 556 nm. The results confirm that the emulsion has long-term stability.

### Example 7

This experiment confirmed that emulsions containing a high concentration of alcohol are stable over time.

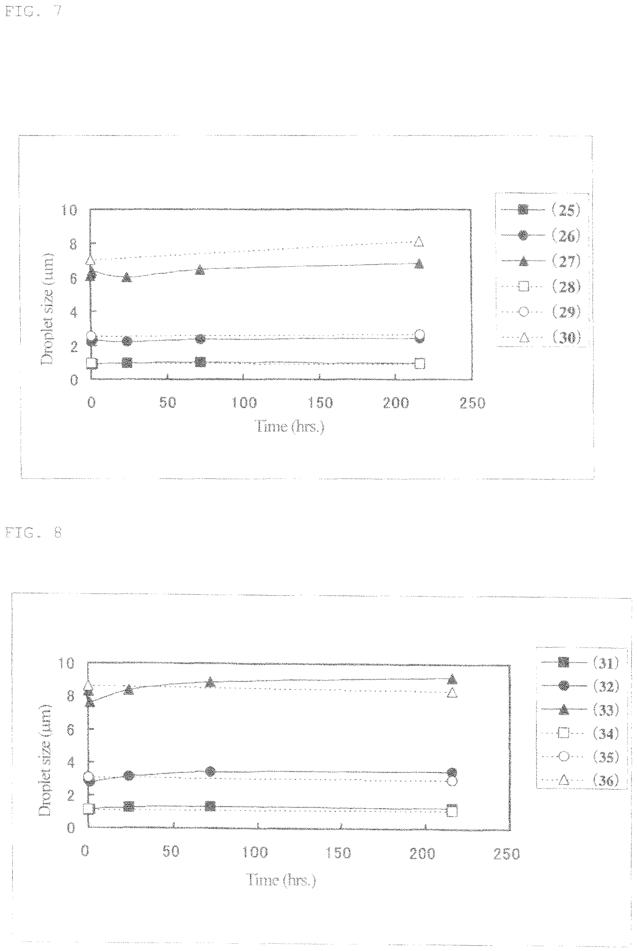

More specifically, 1 g of hydrogenated castor oil with 50 moles of polyoxyethylene added thereto ("HCO-50", product of Nikko Chemicals) was added to 9.5 g of iodinated poppyseed oil fatty acid ethyl ester "Lp" ("Lipiodol Ultra-Fluid", product of Nihon Schering, K.K.) having a specific gravity 1.1 to prepare an oil phase. 90 g of a 5% wt% aqueous glucose solution containing 1 g of "HCO-60" and 1 g of "Poloxamer Po-188" ("Luturol", product of BASF Takeda Vitamin K.K.) was prepared as an external aqueous phase. An O/W emulsion was prepared by stirring at 16,000 rpm using a homomixer for 1 minute. Subsequently, 234 g of ethanol (ethanol concentration: 69.9 wt%) was added to 101 g of this emulsion and mixed by inversion. After 5 minutes, 1 hour, 2 days, and 9 days, the droplet size distribution was measured using the laser diffraction/scattering particle size distribution analyzer ("SALD-2000", product of Shimadzu Corporation). FIG.7 shows changes in 10%D (25), 50%D (26) and 90%D (27) of the emulsion with an ethanol concentration of. 69.9 wt%. For comparison, FIG.7 7 shows changes in 10%D (28), 50%D (29) and 90%D (30) of an O/W emulsion prepared not using ethanol (ethanol concentration: 0 wt%).

The results show that as compared with the emulsion with an ethanol concentration of 0 wt%, the changes in 10%D, 50%D, and 90%D of the emulsion with an ethanol concentration of 69.9 wt% were maintained within the range of 30% for up to 9 days, thus confirming that the emulsion is stable.

Likewise, 1 g of hydrogenated castor oil with 40 moles of polyoxyethylene added ("HCO-40") thereto was added to 8 g of soybean oil with a specific gravity of 0.9 to prepare an oil phase. 90 g of a 5% wt% aqueous glucose solution containing 1 g of "HCO-60" and 1 g of "Poloxamer Po-188" was prepared as an external aqueous phase. An O/W emulsion was prepared by stirring at 16,000 rpm using a homomixer for 1 minute. Subsequently, 234 g of ethanol (ethanol concentration: 70.1 wt%) was added to 100 g of this emulsion and mixed by inversion. The droplet size distribution was measured in the same manner as above. FIG.8 shows changes in 10%D (31), 50%D (32) and 90%D (33) of the emulsion with an ethanol concentration of 70.1 wt%; and changes in 10%D (34), 50%D (35) and 90%D (36) of an emulsion with an ethanol concentration of 0 wt%.

The results show that as compared with the emulsion with an ethanol concentration of 0 wt%, the changes in 10%D, 50%D, and 90%D of the emulsion with an ethanol concentration of 70.1 wt% were maintained within the range of 30% for up to 9 days, thus confirming that the emulsion is stable.

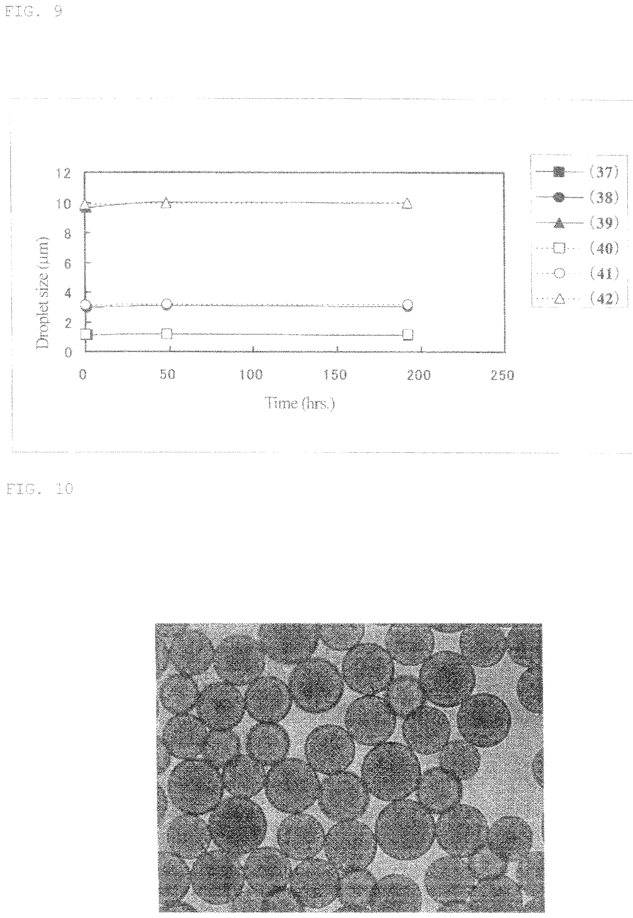

Likewise, 1 g of polyglycerin condensed ricinoleic acid ester ("PGCR") was added to 8 g of soybean oil with a specific gravity of 1.0 to prepare an oil phase. 90 g of a 5% wt% aqueous glucose solution containing 1 g of "HCO-60" and 1 g of "Poloxamer Po-188" was prepared as an external aqueous phase. An O/W emulsion was prepared by stirring at 16,000 rpm using a homomixer for 1 minute. Subsequently, 234 g of ethanol (ethanol concentration: 69.9 wt%) was added to 101 g of this emulsion and mixed by inversion. After 5 minutes, 1 hour, 2 days, and 9 days, the droplet size distribution was measured using the laser diffraction/scattering particle size distribution analyzer ("SALD-2000", product of Shimadzu Corporation). FIG.9 shows changes in 10%D (37), 50%D (38) and 90%D (39) of the emulsion with an ethanol concentration of 69.9 wt%. For comparison, FIG.9 shows changes in 10%D (40), 50%D (41) and 90%D (42) of an O/W emulsion prepared not using ethanol (ethanol concentration: 0 wt%).

The results show that as compared with the emulsion with an ethanol concentration of 0 wt%, the changes in 10%D, 50%D, and 90%D of the emulsion with an ethanol concentration of 69.9 wt% were maintained within the range of 30% for up to 9 days, thus confirming that the emulsion is stable.

The above results confirm that when an emulsifier suitable for the preparation of an emulsion with tolerance to alcohol, such as a polyoxyethylene hydrogenated castor oil "HCO", is added to one of the oil and external aqueous phases, an emulsion with tolerance to alcohol can be produced, regardless of the type of oil used, the kind of emulsifier added to the other phase, and a high ethanol concentration.

### Example 8

In Reference Examples 1 to 4 and Examples 6 and 7, alcohol-containing emulsions were produced by preparing an emulsion and then adding an alcohol thereto. In Example 8, an aqueous phase starting material or oil phase starting material containing a monohydric alcohol or a solution in a monohydric alcohol as a solvent was prepared and then formed into an emulsion to investigate whether or not an emulsion with tolerance to alcohol can be obtained.

Water, ethanol, and "HCO-60" were blended in the proportions shown in Table 5 to prepare external aqueous phases. The emulsifier concentration in each external aqueous phase was 5 wt% or 20 wt%, and the ethanol concentration was 0 to 83 wt%, based on the total weight of the O/W emulsion. While 11 g of soybean oil was added gradually to 89 g of each external aqueous phase, the resulting mixture was stirred at 16,000 rpm for emulsification using a homomixer for 1 minute to prepare an O/W emulsion at room temperature.

**Table 5 A Composition List of O/W Emulsion for Investigating Emulsion Preparation Processes**

| | | | | | | | | | | | |
|---|---|---|---|---|---|---|---|---|---|---|---|
| Liquid phase | Soybean oil | wt% | 9* | 10* | 10 | 10 | 10 | 11 | 11 | 11 | 11* |
| External water phase | HCO-60 | wt% | 5 | 5 | 5 | 5 | 5 | 5 | 5 | 5 | 5 |
| | Water | wt% | 86 | 47 | 43 | 39 | 30 | 20 | 10 | 5 | 0 |
| | Ethanol | wt% | 0 | 38 | 42 | 46 | 55 | 64 | 74 | 79 | 83 |
| Total (%) | | | 100 | 100 | 100 | 100 | 100 | 100 | 100 | 100 | 100 |

| | | | | | | | | | | | |
|---|---|---|---|---|---|---|---|---|---|---|---|
| * Reference Example | | | | | | | | | | | |

One hour and 24 hours after the preparation of the emulsions, the emulsion droplet size distributions were measured using the laser diffraction/scattering particle size distribution analyzer. More specifically, the droplet size distributions of the emulsions produced under the different conditions as shown above confirm that the changes in 10%D, 50%D and 90%D of the emulsions with an ethanol concentration of 64 wt% or less, as observed 1 hour and 24 hours after the preparation were maintained within 30%, and even the emulsions with an ethanol concentration of higher than 64 wt% were maintained as emulsions. More specifically, the results confirm that an emulsion with tolerance to alcohol can be obtained regardless of the production process, i.e., whether the emulsion is produced by preparing an aqueous phase starting material or oil phase starting material containing a monohydric alcohol or a solution in a monohydric alcohol as a solvent beforehand and then producing an emulsion therefrom, or by preparing an emulsion and then adding a monohydric alcohol thereto as in Reference Examples 1 to 4 and Examples 6 and 7.

### Reference Example 9

To confirm that an emulsion with a water concentration of 0 wt%, i.e., an emulsion containing a monohydric alcohol alone or an emulsifier-containing monohydric alcohol alone as an external aqueous phase can be produced, an emulsion was prepared using ethanol containing an emulsifier.

More specifically, an external aqueous phase was prepared by dissolving 53 g of "HCO-60" in 36 g of ethanol. While 11 g of soybean oil was added gradually to 89 g of the external aqueous phase, the resulting mixture was stirred at 16,000 rpm using a homomixer for 1 minute to yield an O/W emulsion at room temperature. The ethanol concentration was 36 wt%, based on the total weight of the O/W emulsion.

Immediately after and 24 hours after the preparation of the emulsion, the state of the emulsion was observed. The results confirm that the state of the emulsion, in spite of the lack of water, was maintained even 24 hours after the preparation.

### Reference Example 10

To confirm that alcohol-containing multiphase emulsions can be produced, ethanol-containing W/O/W and O/W/O multiphase emulsions were prepared.

10 mg of fluoresce-in-isothiocyanatedextran (product of Sigma) was dissolved in 490 mg of distilled water, and dispersed in 0.72 g of a soybean oil containing 0.2 g of PGCR using a homomixer to prepare a W/O emulsion. The emulsion was then forced through a porous glass membrane (product of SPG Techno Co.) with a pore size of 15 µm to yield a W/O/W emulsion comprising a 5 wt% aqueous glucose solution containing 0.05 g of HCO-60 as an external aqueous phase. Subsequently, 3.12 g of ethanol was added to the emulsion to yield a W/O/W emulsion with an ethanol concentration of 34.6 wt%.

This emulsion was observed under an optical microscope. The results show that no droplet coalescence occurred, thus confirming that the emulsion was stable even 20 hours after the preparation. FIG.10 shows an optical microscopic photograph taken 20 hours after the preparation.

Next, an external aqueous phase was prepared by dissolving 0.2 g of HCO-60, 3.77 g of water and 3.12 g of ethanol. Subsequently, 1.8 g of soybean oil was dispersed in the external aqueous phase using a homomixer to prepare 8.89 g of an O/W emulsion. This O/W emulsion was dispersed with stirring in an oil phase prepared by mixing 14.4 g of a soybean oil and 4 g of PGCR, thus yielding an O/W/O emulsion with an ethanol concentration of 11.4 wt%.

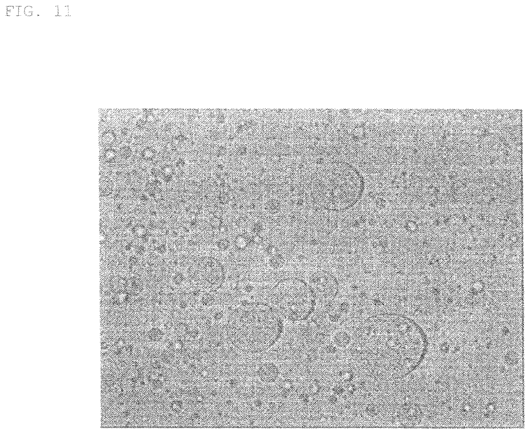

This emulsion was observed under an optical microscope in the same manner as described above. The results show that no oil-water separation occurred even 1 hour after the preparation, thus confirming that the emulsion was stable. FIG.11 shows an optical microscopic photograph taken 1 hour after the preparation.

## Claims

1. An emulsion with tolerance to alcohol comprising a monohydric alcohol; and an emulsifier;
(1) the content of the monohydric alcohol being 40 to 80 wt%, based on the total weight of the emulsion;
(2) the content of the emulsifier being 0.1 to 50 wt%, based on the total weight of the emulsion; and
(3) the emulsion being in the form of an O/W emulsion, W/O/W emulsion, O/W/O emulsion, or S/O/W emulsion,
wherein the emulsifier comprises at least one emulsifier selected from the group consisting of 1) polyoxyethylene hydrogenated castor oils, and 2) polyoxyethylene castor oils.

2. The emulsion with tolerance to alcohol according to Claim 1 wherein the monohydric alcohol accounts for 99.99 to 100 wt% of the total alcohol content of the emulsion with tolerance to alcohol.

3. A process for preparing an emulsion with tolerance to alcohol comprising: (1) a first step of adding an emulsifier to at least either of an aqueous phase starting material or an oil phase starting material; (2) a second step of mixing the aqueous phase starting material and the oil phase starting material to form a precursor emulsion; and (3) a third step of mixing the precursor emulsion with a monohydric alcohol wherein the content of the monohydric alcohol is 40 to 80 wt%, based on the total weight of the emulsion, and wherein the emulsifier comprises at least one emulsifier selected from the group consisting of 1) polyoxyethylene hydrogenated castor oils, and 2) polyoxyethylene castor oils.

4. The process according to Claim 3, further comprising a step of removing the alcohol from the emulsion obtained in step (3).

5. The process according to Claim 3, further comprising a step of forcing the emulsion obtained in step (3) through a porous membrane to reduce the droplet size of the emulsion.

## Patentansprüche

1. Eine alkoholbeständige Emulsion, umfassend einen einwertigen Alkohol; und einen Emulgator;
(1) wobei der Gehalt des einwertigen Alkohols 40 bis 80 Gewichtsprozent beträgt, bezogen auf das Gesamtgewicht der Emulsion;
(2) wobei der Gehalt des Emulgators 0,1 bis 50 Gewichtsprozent beträgt, bezogen auf das Gesamtgewicht der Emulsion; und
(3) wobei die Emulsion in Form einer Öl-in-Wasser Emulsion, Wasser-in-Öl-in-Wasser Emulsion, Öl-in-Wasser-in-Öl Emulsion, oder Feststoff-in-Öl-in-Wasser Emulsion vorliegt,
wobei der Emulgator mindestens einen Emulgator, ausgewählt aus der Gruppe bestehend aus 1) Polyoxyethylen-hydrierten Rizinusölen, und 2) Polyoxyethylen-Rizinusölen, umfasst.

2. Die alkoholbeständige Emulsion gemäß Anspruch 1, wobei der einwertige Alkohol 99,99 bis 100 Gewichtsprozent des Gesamtalkoholgehalts der alkoholbeständigen Emulsion ausmacht.

3. Ein Verfahren zur Herstellung einer alkoholbeständigen Emulsion, umfassend: (1) einen ersten Schritt des Hinzufügens eines Emulgators zu mindestens einem Ausgangsmaterial einer wässrigen Phase und/oder einem Ausgangsmaterial einer Ölphase; (2) einen zweiten Schritt des Mischens des Ausgangsmaterials der wässrigen Phase und des Ausgangsmaterials der Ölphase, um eine Vorläuferemulsion zu bilden; (3) einen dritten Schritt des Mischens der Vorläuferemulsion mit einem einwertigen Alkohol, wobei der Gehalt des einwertigen Alkohols 40 bis 80 Gewichtsprozent, bezogen auf das Gesamtgewicht der Emulsion, beträgt, und wobei der Emulgator mindestens einen Emulgator, ausgewählt aus der Gruppe bestehend aus 1) Polyoxyethylen-hydrierten Rizinusölen, und 2) Polyoxyethylen-Rizinusölen, umfasst.

4. Das Verfahren gemäß Anspruch 3, weiterhin umfassend einen Schritt des Entfernens des Alkohols aus der in Schritt (3) erhaltenen Emulsion.

5. Das Verfahren gemäß Anspruch 3, weiterhin umfassend einen Schritt des Hindurchtreibens der in Schritt (3) erhaltenen Emulsion durch eine poröse Membran, um die Tröpfchengröße der Emulsion zu reduzieren.

## Revendications

1. Emulsion présentant une tolérance à l'alcool comprenant un alcool monohydrique ; et un émulsifiant ;
(1) la teneur en alcool monohydrique étant de 40 à 80 % en poids, sur la base du poids total de l'émulsion ;
(2) la teneur en émulsifiant étant de 0,1 à 50 % en poids, sur la base du poids total de l'émulsion ; et
(3) l'émulsion étant sous la forme d'une émulsion huile/eau (O/W), d'une émulsion eau/huile/eau (W/O/W), d'une émulsion huile/eau/huile (O/W/O) ou d'une émulsion solide/huile/eau (S/O/W),
dans laquelle l'émulsifiant comprend au moins un émulsifiant choisi parmi le groupe constitué par 1) des huiles de castor hydrogénées de polyoxyéthylène et 2) des huiles de castor de polyoxyéthylène.

2. Emulsion présentant une tolérance à l'alcool selon la revendication 1, dans laquelle l'alcool monohydrique compte pour 99,99 à 100 % en poids de la teneur en alcool totale de l'émulsion présentant une tolérance à l'alcool.

3. Procédé pour préparer une émulsion présentant une tolérance à l'alcool comprenant : (1) une première étape d'ajout d'un émulsifiant à au moins soit un produit de départ en phase aqueuse, soit un produit de départ en phase huileuse ; (2) une deuxième étape consistant de mélange du produit de départ en phase aqueuse et du produit de départ en phase huileuse de manière à former une émulsion précurseur ; et (3) une troisième étape de mélange de l'émulsion précurseur avec un alcool monohydrique, dans lequel la teneur en alcool monohydrique est de 40 à 80 % en poids, sur la base du poids total de l'émulsion, et dans lequel l'émulsifiant comprend au moins un émulsifiant choisi parmi le groupe constitué par 1) des huiles de castor hydrogénées de polyoxyéthylène et 2) des huiles de castor de polyoxyéthylène.

4. Procédé selon la revendication 3, comprenant en outre une étape consistant à enlever l'alcool de l'émulsion obtenue à l'étape (3).

5. Procédé selon la revendication 3, comprenant en outre une étape consistant à forcer l'émulsion obtenue à l'étape (3) au travers d'une membrane poreuse de manière à réduire la dimension des gouttelettes de l'émulsion.
